# EUROPÄISCHE PATENTSCHRIFT

(11) **EP 1 616 594 B1**
(45) Veröffentlichungstag und Bekanntmachung des Hinweises auf die Patenterteilung: **24.01.2007**
(21) Anmeldenummer: 04016627.4
(22) Anmeldetag: 15.07.2004
(51) Int. Cl.: A61M 25/06, A61M 25/02

(54) **Punktionsanordnung mit flexiblem Verweilschlauch zum Anschliessen an eine medizinische Versorgungsleitung**
Punctionsystem with a flexible permanent tube to link to a medical distribution tube
Système de ponction avec un tube flexible permanent raccordé à un système d'alimentation médical

(43) Veröffentlichungstag der Anmeldung: 18.01.2006
(73) Patentinhaber: Clinico GmbH, 36251 Bad Hersfeld (DE)
(72) Erfinder: Heinzerling, Jörg, 36251 Bad Hersfeld (DE); Bölinger, Simone, Dr., 41239 Mönchengladbach (DE); Csincsura, Roland, 99817 Eisenach (DE)
(74) Vertreter: von Eichel-Streiber, Caspar

(56) Entgegenhaltungen:
- EP-A- 1 362 607
- DE-U- 29 503 099
- US-A- 5 545 143
- US-A- 5 713 872
- US-A1- 2003 149 404

## Beschreibung

Die Erfindung betrifft eine Punktionsanordnung mit einem flexiblen Verweilschlauch zum Anschließen an eine medizinische Versorgungsleitung mit einem an einer Einstichstelle auf der Hautoberfläche eines Patienten fixierbaren Katheteranschlussstück (Hub), welcher einen Anschluss zum Anlegen der Versorgungsleitung und eine Austrittsöffnung für den flexiblen Verweilschlauch aufweist, und mit einem eine Punktionsnadel (Stilett) aufweisenden Nadelhalter, wobei der flexible Verweilschlauch im Inneren des Katheteranschlussstückes angeordnet und mit einem Abgabeende aus der Austrittsöffnung heraus geführt ist, wobei das dem Abgabeende gegenüberliegende Ende des flexiblen Verweilschlauches im Inneren des Katheteranschlussstückes im Bereich des Anschlusses liegt und von der Umgebung des Katheteranschlussstückes durch eine Dichtung (Septum) getrennt ist und wobei der Nadelhalter die in einen Durchflusskanal des Verweilschlauches einführbare Punktionsnadel an einem dem spitzen bzw. scharfen Punktionsende derselben gegenüberliegenden Ende fixiert und in einer Ausgangstellung der Punktionsanordnung so an dem Anschluss des Katheteranschlussstückes von diesem lösbar angeordnet ist, dass die Punktionsnadel die Dichtung durchdringt und bis über das Abgabeende hinaus in dem Durchflusskanal des Verweilschlauches geführt ist.

Eine grundsätzlich ähnliche Punktionsanordnung ist in der US 5,545,143 offenbart. Allerdings ist die Punktionsnadel bei der hier offenbarten Punktionsanordnung nicht etwa entlang des gesamten flexiblen Verweilschlauches bis zu dem Anschluss geführt, sondern durchragt das Katheteranschlussstück "nach oben" in einer Position mitten im flexiblen Verweilschlauch. Durch diese Konstruktion ergibt sich ein hinsichtlich der Sterilität problematischer Totraum im dem Abgabeende gegenüberliegenden, hinteren Abschnitt des flexiblen Verweilschlauches.

In der Medizin werden in verschiedenen Bereichen zur Verabreichung von in Flüssigkeit gelösten oder suspendierten Medikamenten Katheter verwendet, durch die über eine Punktionsstelle die Medikation direkt in das Gewebe oder eine Blutbahn des Patienten verabreicht wird. Dabei muss zum Anlegen des Katheters zunächst ein Zugang zum Gewebe bzw. der Blutbahn des Patienten durch Punktion geschaffen werden. Hierfür sind verschiedentliche Punktionsanordnungen bekannt.

Punktionsanordnungen gemäß der eingangs genannten Art sind insbesondere für die eigenständige Anwendung durch den Patienten selbst, beispielsweise für die Insulinverabreichung mit Hilfe einer Insulinpumpe, gedacht.

Neben den bekannten Punktionsanordnungen mit flexiblem Verweilschlauch existieren vergleichbare Produkte, die anstelle eines solchen Verweilschlauches eine Hohlnadel aus Stahl oder einem vergleichbaren Metall aufweisen, durch welche die Medikation verabreicht wird.

Bei Punktionsanordnungen mit flexiblem Verweilschlauch und einer Punktionsnadel wird zugleich mit dem Aufsetzen des Katheteranschlussstückes die Hautoberfläche und das darunter liegende Gewebe des Patienten durch die Punktionsnadel punktiert und in diesem Zuge der flexible Verweilschlauch in das Gewebe eingebracht. Als Einstichpunkt für die Punktionsanordnung wird dabei häufig der Bauchbereich des Patienten gewählt. Nach dem Einstechen wird die Punktionsnadel zurückgezogen und entfernt, und es verbleibt in der Punktionsstelle lediglich der Verweilschlauch, der zuvor an eine an das Katheteranschlusstück angekoppelte Versorgungsleitung, somit an eine Medikamentenversorgung, angeschlossen wurde.

Bei einer Art von bekannten Punktionsanordnungen dieser Art liegt die Austrittsöffnung auf einer in Gebrauchsstellung auf der Patientenoberfläche aufliegenden Unterseite des Katheteranschlussstückes und ein Abgabeende des flexiblen Verweilschlauches gegenüber liegendes Anschlussende und das einer scharfen bzw. spitzen Ende einer Punktionsnadel gegenüberliegende Ende derselben sind an unterschiedlichen Öffnungen aus dem Katheteranschlussstück herausgeführt. Beide dieser Öffnungen sind dabei mit einer Dichtung gegenüber der Umgebung abgedichtet.

Eine Punktionsanordnung der eingangs genannten Art wird von der Firma Disetronic Medical Systems AG, Burgdorf, Schweiz, unter der Bezeichnung "Disetronic®Tender" vertrieben. Bei dieser Punktionsanordnung ist die Austrittsöffnung für den flexiblen Verweilschlauch seitlich an dem Katheteranschlussstück angeordnet. Durch den im Innern des Katheteranschlussstücks geführten Verweilschlauch erstreckt sich zum Punktieren eine in einem Nadelhalter gehaltene Hohlnadel als Punktionsnadel. Die Nadel erstreckt sich im wesentlichen gerade und tritt an der Seite aus dem Katheteranschlussstück nahezu waagerecht aus. Zum Anbringen des Katheters muss der Patient die Punktionsnadel mit dem vorstehenden Ende des Verweilschlauches nach Augenmaß in einem Winkel von 30° bis 45° in die Haut einstechen, diese dann nach hinten aus dem Verweilschlauch herausziehen und dann das Katheteranschlussstück um die etwa 30° bis 45° verkippen und flach auf der Hautoberfläche befestigen. Dabei wird auch das nun im Körper befindliche Ende des flexiblen Verweilschlauches umgebogen.

Zwar ist bei dieser Punktionsanordnung eine zusätzliche Dichtung nicht erforderlich, es ergibt sich aber die für die oben genannten Punktionsanordnungen ebenfalls bestehende Problematik, dass vor dem Einsatz einer solchen Punktionsanordnung das freie Volumen des Verweilschlauches entlüftet bzw. mit dem zu verabreichenden Medikament geflutet werden muss. Dazu muß die durch die Dichtung in das Austrittsende des Verweilschlauches zum Schaffen der Punktion eingeführte Punktionsnadel als Hohlnadel mit einem seitlich angeordneten Entlüftungsschliff bzw. einer Entlüftungsbohrung ausgeführt sein, damit die Entlüftung durch deren Hohlraum bzw. Hohlkanal erfolgen kann. Da Hohlnadeln in ihrem minimalen Durchmesser dadurch beschränkt sind, daß der Hohlraum bzw. der Hohlkanal der Nadel noch von einem Fluid durchströmt werden kann und das diesen Hohlraum umgebende Material stabil genug sein muß, nicht zu brechen, ist zwangsläufig der minimale Außendurchmesser des Verweilschlauches auf einen größeren Durchmesser beschränkt als die in dieser geführte Hohlnadel zur Punktion. Aufgrund dieses dadurch vergleichsweise großen Verweilschlauches (üblich sind hier Außendurchmesser von 0,5 bis 0,7 mm) werden diese von den Patienten als störend bzw. sogar schmerzhaft empfunden. Auch hinterlassen diese Punktionsanordnungen nach dem Entfernen des Katheters im Durchmesser größere Verletzungen, die entsprechend langsamer heilen verbunden mit einer Infektionsgefahr. Nicht zuletzt deshalb werden Punktionsanordnungen der eingangs genannten Art trotz ihrer ansonsten gegenüber den Punktionsanordnungen mit fester Hohlnadel aus Metall vorhandenen Vorteile (z. B. keine Probleme mit Nickelunverträglichkeit, keine starre Nadel, die bei einem Anstoßen an den Katheter Schmerzen verursacht) nicht in dem gewünschten bzw. möglichen Maße von den Patienten akzeptiert. Zudem macht die in einem zusätzlichen Schritt erforderliche Entlüftung insbesondere für einen Patienten in der Selbstanwendung das Ansetzen bzw. die Bedienung der Punktionsanordnung kompliziert und birgt bei einer nicht sachgerechten Handhabung Risiken.

Die bekannte Punktionsanordnung Disetronic®Tender ist darüber hinaus in der Handhabung kompliziert (Winkelwahl) und es besteht die Gefahr, dass bei Fehlbedienung (zu großer Winkel) das Austrittsende des Verweilschlauches abgeklemmt und damit für einen Medikamentendurchfluss undurchlässig wird oder gar die als Hohlnadel ausgeführte Punktionsnadel im Körper des Patienten abbricht.

Der vorliegenden Erfindung liegt daher die Aufgabe zugrunde, eine Punktionsanordnung der eingangs genannten Art dahingehend zu verbessern, dass bei einer vereinfachten Handhabung ihr Verweilschlauch einen verglichen mit den bekannten Verweilschläuchen geringeren Außendurchmesser aufweisen kann.

Zur Lösung dieser Aufgabe wird für eine Punktionsanordnung der eingangs genannten Art vorgeschlagen, dass die Austrittsöffnung auf einer in Gebrauchsstellung auf der Patientenoberfläche aufliegenden Unterseite des Katheteranschlussstückes angeordnet ist und dass die Punktionsnadel durchgangslos gefertigt ist..

Erfindungsgemäß ist demnach die entlang des ganzen Weges durch den flexiblen Verweilschlauch bis zum Anschluss, an dem auch der flexible Verweilschlauch endet, und von dort durch dieselbe Dichtung, mit der auch der rückwärtige Anschluss des flexiblen Verweilschlauches gegenüber der Umgebung abgedichtet ist, hindurchgeführte Punktionsnadel anders als beim vorbekannten Stand der Technik durchgangslos gefertigt. Auf diese Weise füllt in der Ausgangsstellung die Punktionsnadel den Innenraum des flexiblen Verweilschlauches aus, es gibt kein zusätzliches Volumen, in welchem eine Luftblase steht, die zu entlüften ist, oder welches zu fluten ist. Nach der Punktion kann die Punktionsnadel axial aus dem Verweilschlauch und aus dem Katheteranschlussstück herausgezogen werden. Durch den dadurch entstehenden Unterdruck und Saugeffekt wird der flexible Verweilschlauch automatisch nahezu vollständig mit Körperflüssigkeit (Gewebsflüssigkeit, Blut) gefüllt. Ein separates Entlüften bzw. Fluten durch eine hohle Punktionsnadel kann entfallen. Die Punktionsnadel kann bspw. aus Vollmaterial, in Sandwichbauweise oder mit einseitig bzw. beidseitig verschlossenen Enden, gefertigt und damit im Außendurchmesser wesentlich kleiner gestaltet werden. Damit kann auch der Außendurchmesser des Verweilschlauches reduziert werden; er kann beispielsweise bei 0,4 mm oder kleiner liegen. Neben dem verringerten Außendurchmesser des Verweilschlauches und der nicht mehr erforderlichen separaten Entlüftung liegt ein weiterer Vorteil der erfindungsgemäßen Bauform darin, dass verglichen mit einigen aus dem Stand der Technik bekannten Punktionsanordnungen eine Dichtung eingespart werden kann. Zudem bedingt der verringerte Außendurchmesser des Verweilschlauches ein deutlich verringertes Volumen in dem Schlauch, welches nach dem Anbringen der Punktionsanordnung anfänglich mit Körperflüssigkeit gefüllt ist und anfangs beim Ansetzen einer Versorgung (bspw. einer Versorgungsleitung von bzw. zu einer Insulinpumpe) verdrängt werden muß und so insbesondere bei feinen Dosierungen die Anfangsdosen verfälscht. Nicht zuletzt ist eine durchgangslose Nadel kostengünstiger herzustellen als eine Hohlnadel, die zum Abführen der Luft noch einen zusätzlichen Schliff bzw. eine Bohrung in den im Inneren liegenden Kanal erfordert. Eine Vergrößerung des Innendurchmessers des Verweilschlauches bedingt schließlich auch eine geringere Angriffsfläche für Zellwachstum und damit längere Liegezeiten verglichen mit der eingangs genannten Anordnung mit Stahlhohlnadel.

Dadurch, dass die Austrittsöffnung ferner auf der Unterseite des Katheteranschlussstückes angeordnet ist, kann die Punktionsanordnung vom Patienten einfach gehandhabt werden. Er muss diese nur auf die Hautoberfläche aufsetzen und zum Punktieren andrücken, ohne einen Einstechwinkel beachten zu müssen.

Selbstverständlich kann der flexible Verweilschlauch der erfindungsgemäßen Punktionsanordnung im Bedarfsfall auch in einem größeren Durchmesser ausgeführt werden; er kann auch abhängig vom Anwendungsgebiet in verschiedenen aus dem Katheteranschlussstück vorstehenden Längen gebildet sein, um in unterschiedliche Gewebeschichten vorzudringen.

Der flexible Verweilschlauch kann vorzugsweise aus Kunststoff bestehen, ohne auf dieses Material beschränkt zu sein. Andere flexible Materialien sind ebenfalls denkbar. Bei der Auswahl des Materials wird selbstverständlich auf dessen Verträglichkeit Rücksicht genommen werden.

Vorteilhafte Weiterbildungen der erfindungsgemäßen Punktionsanordnung sind in den Ansprüchen 2 bis 10 gekennzeichnet.

Eine im wesentlichen flache Bauform der Punktionsanordnung, wie dies in Anspruch 2 bestimmt ist, ist insbesondere bei solchen Punktionsanordnungen von Vorteil, die bspw. von Insulinpatienten "im täglichen Leben" genutzt werden. Da diese sich mit angesetzten Punktionsanordnungen und angefügten Versorgungsleitungen normal bewegen sollen, ist ein im wesentlichen flach ausgeführtes Katheteranschlussstück weit weniger störend, da es bspw. deutlich weniger dazu neigt, an Kleidungsstücken hängen zu bleiben. Ein Anstoßen an einem weit vorstehenden Katheteranschlussstück kann einerseits schmerzhaft sein, andererseits sogar zu einem Ablösen des Katheteranschlussstückes und somit des Verweilschlauches führen, so dass zur Sicherstellung der Medikamentenversorgung ein neuer Zugang gelegt werden müsste. Bei der Ausgestaltung der Punktionsanordnung gemäß Anspruch 2 wird selbstverständlich auch die Punktionsnadel, welche sich in der Ausgangsstellung der Punktionsanordnung durch den Verweilschlauch hindurch erstreckt entsprechend dem Verlauf des Verweilschlauches gebogen verlaufen. Mögliche Winkel des Abgabeendes des Verweilschlauches zu dem Katheteranschlussstück liegen prinzipiell bei allen Winkeln zwischen 0° und 90°, wobei Winkel von 30° und mehr bevorzugt werden.

Eine Punktionsanordnung, wie sie in Anspruch 3 definiert ist, bietet den Vorteil, dass nach dem Punktieren und Aufsetzen des Katheteranschlussstückes die Punktionsnadel einfach und sicher aus dem Verweilschlauch zurückgezogen werden kann. Durch einfaches Angreifen und Ziehen an dem hinteren Abschnitt des Nadelhalters wird achsparallel zur Mittelachse des flexiblen Verweilschlauches eine Kraft auf die Punktionsnadel ausgeübt, welche ein Rückziehen derselben aus dem Verweilschlauch bewirkt, ohne dass seitliche Kräfte auf den Verweilschlauch ausgeübt werden, welche ein Verrutschen oder Lösen des Verweilschlauches aus der Punktionsstelle bewirken könnten. Nach dem Herausziehen der Punktionsnadel aus dem Verweilschlauch kann der Nadelhalter von dem Anschluss des Katheteranschlussstückes getrennt, entnommen und entsorgt werden.

Für ein besseres Handhaben beim Abtrennen des Nadelhalters weist dieser gemäß Anspruch 6 vorzugsweise ein Griffstück auf.

Wurde die Punktionsnadel aus dem Innern des Verweilschlauches entfernt, dichtet die Dichtung das Volumen im Innern des Verweilschlauches sicher gegen äußere Einflüsse ab, bis ein Anschlussgegenstück einer Versorgungsleitung mit einem geeigneten Element, bspw. einer Nadel, diese Dichtung durchsticht und eine in Flüssigkeit gelöste oder suspendierte Medikamentenversorgung zuführt.

Ist die gemäß Anspruch 3 ausgebildete Punktionsanordnung zusätzlich wie in Anspruch 4 angegeben gestaltet, können nach dem Herausziehen der Punktionsnadel aus dem Inneren des Verweilschlauches durch Strecken der Schenkel des flexiblen Abschnittes des Nadelhalters diese Schenkel durch Verrasten gegeneinander gesichert werden, wodurch ein Wiedereindringen der Punktionsnadel in den flexiblen Verweilschlauch verhindert wird. Zugleich bleibt die Punktionsnadel so in einer zurück gezogenen Position, so dass sie auch bei der Entsorgung des Nadelhalters keine Gefährdung für den Patienten oder andere Personen darstellen kann.

Um eine Gefährdung durch die Punktionsnadel bei von dem Katheteranschlussstück entferntem Nadelhalter weiterhin auszuschließe, ist die Punktionsanordnung vorzugsweise wie in Anspruch 5 angegeben ausgebildet. Gemäß dieser Variante liegt die Punktionsnadel mit ihrem Punktionsende nach Gebrauch innerhalb des Nadelhalters, so dass die Gefahr des Stechens für die Patienten oder dritte Personen (immer auch verbunden mit einer Infektionsgefahr) unterbunden ist.

Für ein einfaches Anschließen einer Versorgungsleitung an den Verweilschlauch ist die Dichtung vorzugsweise wie in Anspruch 7 angegeben vorperforiert.

Damit das Katheteranschlussstück auf der Hautoberfläche des Patienten sicher anhaften bleibt, weist es wie in Anspruch 8 vorgesehen vorzugsweise eine selbstklebende Schicht auf einer Unterseite auf. Diese kann bspw. ein auf einen Zellstoff aufgebrachter Klebstofffilm sein und wird vor der Benutzung des Katheters durch eine Schutzfolie abgedeckt sein.

Schließlich ist zu bevorzugen, dass die Punktionsanordnung in einem orginal versiegelten Zustand, wie in Anspruch 9 vorgesehen, eine Schutzkappe aufweist. Auch diese verhindert Verletzungen durch einen unbeabsichtigten Stich der Punktionsnadel vor dem Gebrauch und sorgt weiterhin für einen keimfreien Verweilschlauch.

Eine Doppelbiegung des Verweilschlauches, wie sei in Anspruch 10 angegeben ist, ist günstig für die Fixierung des Verweilschlauches im Katheteranschlussstück, z.B. durch Insertmolding oder Kleben. Ferner reduziert eine solche (grundsätzlich als S-förmig zu bezeichnende) Führung des Verweilschlauches die beim Herausziehen der anfangs durch den Verweilschlauch geführten Punktionsnadel entstehenden Gleitkräfte und verhindert so einen Abbruch der letzterer.

Eine weitere vorteilhafte Ausgestaltung der erfindungsgemäßen Punktionsanordnung liegt darin, daß die Wandung des Verweilschlauches an seinem Abgabeende in der Ausgangsstellung in Richtung zu dem Punktionsende der Punktionsnadel hin abgerundet bzw. abgeflacht ist, gibt es hier keine abrupte Kante, die beim Punktieren bzw. Einführen der Verweilkanüle beim Patienten Schmerzen verursachen kann. Das Abrunden bzw. Abflachen kann bspw. durch Andrücken des Endes der Verweilkanüle an die Punktionsnadel geschehen.

Weitere Vorteile und Merkmale der Erfindung ergeben sich aus der nachfolgenden Beschreibung eines Ausführungsbeispiels im Zusammenhang mit den Figuren. In den Figuren zeigen:
- Fig. 1: eine erfindungsgemäße Punktionsanordnung in der Ausgangsstellung in einer geschnittenen Seitenansicht,
- Fig. 2: die Punktionsanordnung aus Fig. 1 in einer dreidimensionalen Darstellung schräg von oben,
- Fig. 3: die erfindungsgemäße Punktionsanordnung in einer Fig. 1 vergleichbaren geschnittenen Seitenansicht nach der Punktion und dem Zurückziehen der Punktionsnadel,
- Fig. 4: die erfindungsgemäße Punktionsanordnung gemäß Fig. 3 in einer dreidimensionalen Ansicht schräg von oben und
- Fig. 5: das Katheteranschlussstück der erfindungsgemäßen Punktionsanordnung nach Ablösen des Nadelhalters mit dem Anschluss für eine Versorgungsleitung sowie ein schematisch angedeutetes, anschlussbereites Gegenstück einer Versorgungsleitung.

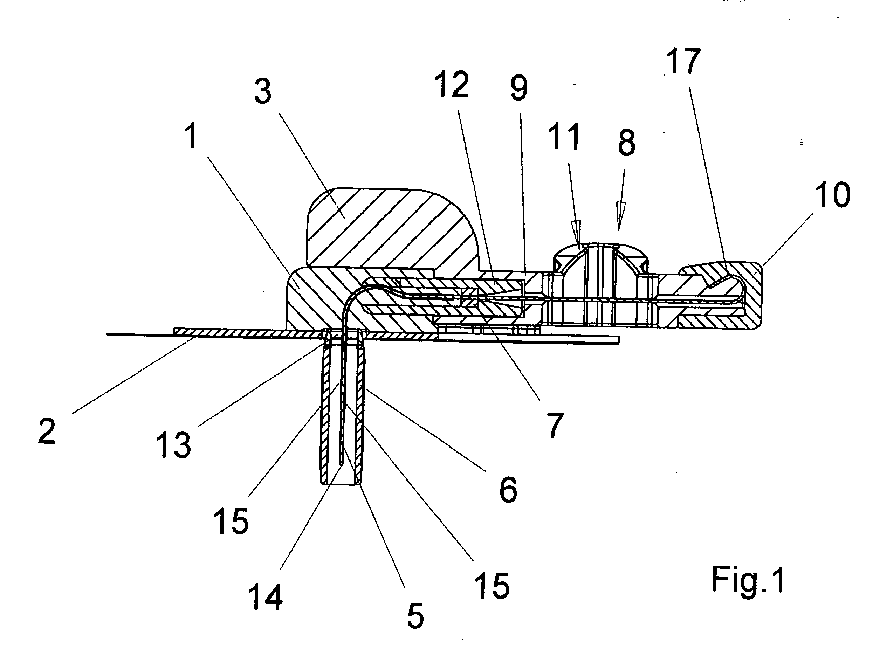

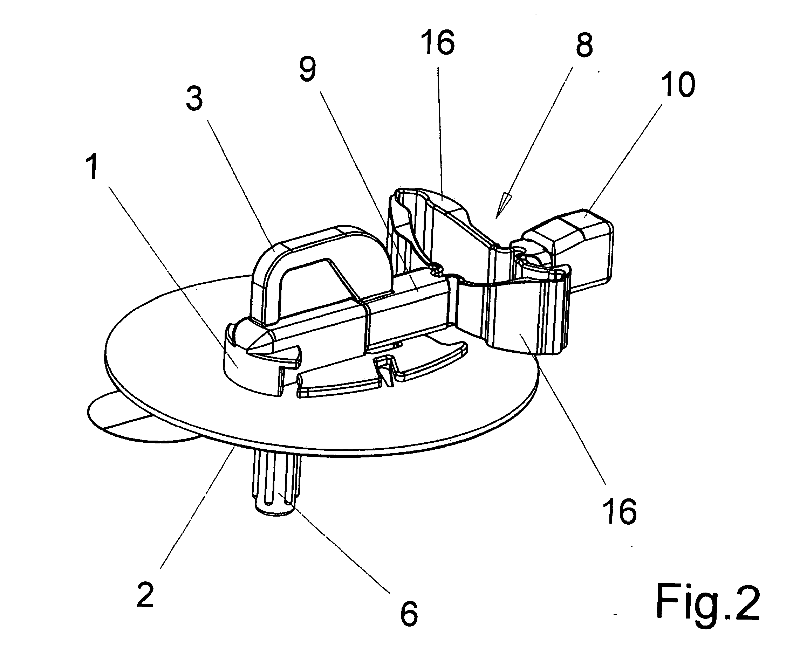

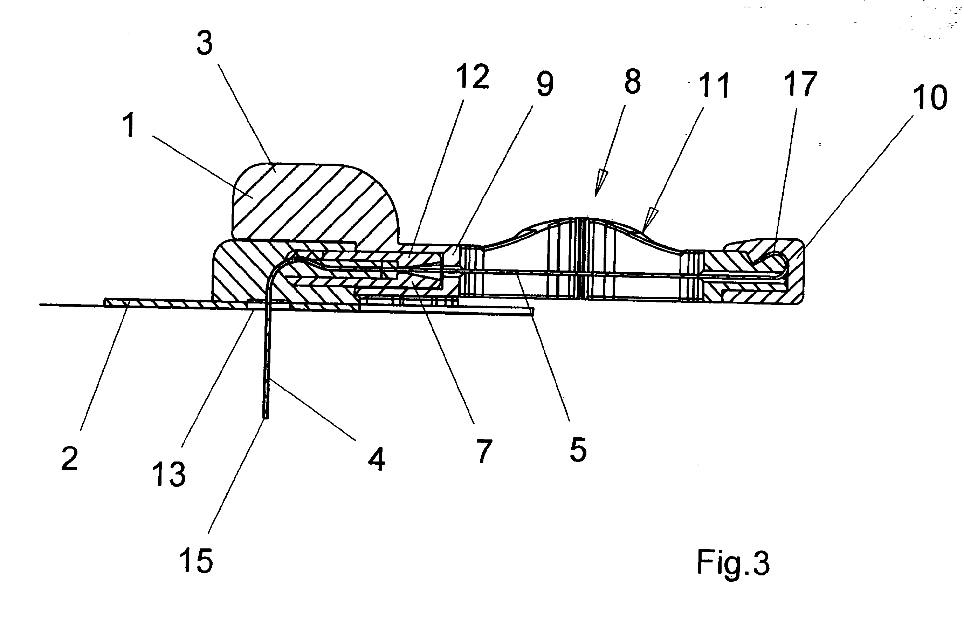

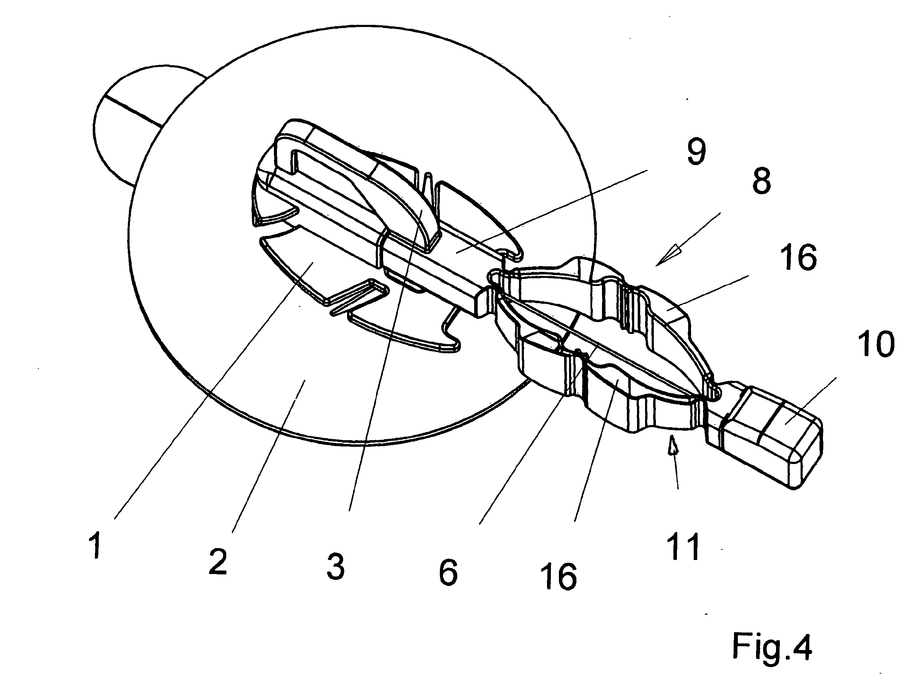

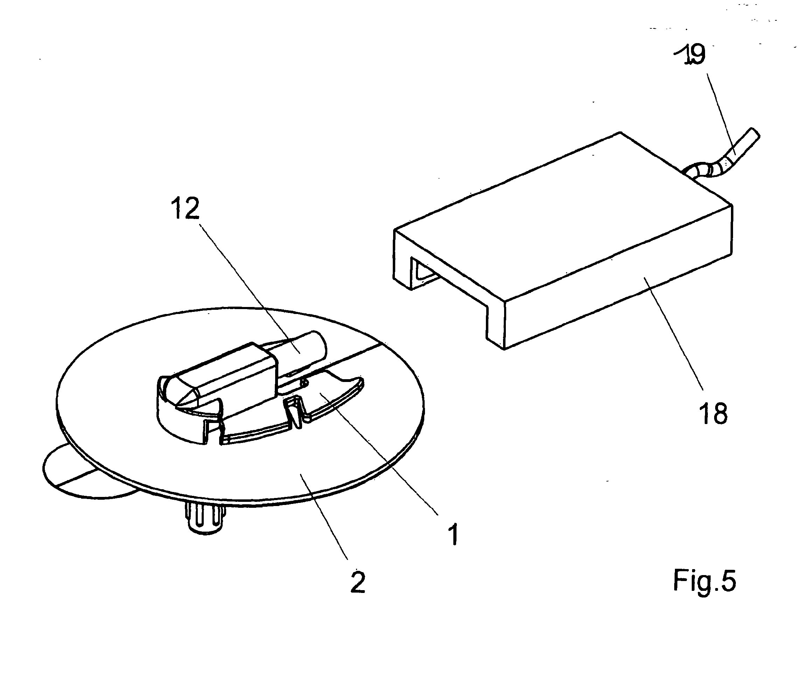

In den Figuren 1 bis 5 ist in verschiedenen Ansichten und verschiedenen Gebrauchsstellungen ein Ausführungsbeispiel einer erfindungsgemäßen Punktionsanordnung gezeigt. Dabei zeigen die Figuren 1 und 2 die Punktionsanordnung in einer Ausgangsstellung vor der Punktion, die Figuren 3 und 4 die Punktionsanordnung in einer Stellung nach der Punktion und nach dem Zurückziehen der Punktionsnadel aus dem Verweilschlauch und Figur 5 das Katheteranschlussstück nach dem Entfernen des Nadelhalters zusammen mit einem anschlussbereiten Gegenstück einer Versorgungsleitung.

Die Punktionsanordnung ist im wesentlichen zweiteilig aufgebaut, aus einem Katheteranschlussstück (Hub) 1 und einem Nadelhalter 8. Das Katheteranschlussstück 1 ist im wesentlichen flach ausgebildet. Das Katheteranschlussstück 1 weist eine Klebeplatte 2 auf mit einem auf deren Unterseite angebrachten selbstklebenden Film, welcher in der in Figuren 1 und 2 gezeigten Ausgangsstellung mit einer Schutzfolie abgedeckt ist. Das Katheteranschlussstück 1 weist ferner einen Anschluss 12 auf, über welchen es zum Gebrauch mit einer medizinischen Versorgungsleitung verbindbar ist. Der Anschluss 12 hat eine im wesentlichen zylinderförmige Außenform und ist mit einer Mittelachse parallel zu der Ebene des flachen Katheteranschlussstückes 1 ausgerichtet. Durch eine Austrittsöffnung 13 auf der im Gebrauch unten liegenden Unterseite des Katheteranschlussstückes 1 ist ein flexibler Verweilschlauch 4 im wesentlichen senkrecht zu der flachen Erstreckungsrichtung des Katheteranschlussstückes 1 aus diesem herausgeführt. In der Ausgangsstellung erstreckt sich eine Punktionsnadel 5 durch den hohlen Innenraum des Verweilschlauches 4 hindurch und durchragt letztere mit einem spitzen bzw. scharfen Punktionsende 14 an dem Abgabeende 15 des Verweilschlauches 4. In der Ausgangsstellung ist dieser Bereich durch eine Schutzkappe 6 abgedeckt, um Verletzungen zu verhindern und eine Verkeimung der Punktionsnadel 5 und des Verweilschlauches 4 zu vermeiden. Der Verweilschlauch 4 ist in den Korpus des Katheteranschlussstückes 1 hineingeführt und dort im Wege einer Doppelbiegung (sozusagen entlang einer S-Form) um etwa 90° umgelenkt, um im wesentlichen mittig entlang der Mittelachse des Anschlusses 12 in diesen hinein und zum Ende desselben hingeführt zu werden. Der Verweilschlauch 4 endet in einer in dem Anschluss 12 angeordneten Dichtung (einem Septum) 7. Die Doppelbiegung dient vor allem dazu, die beim Herausziehen der Punktionsnadel 5 aus dem Verweilschlauch 4 auftretenden Gleitkräfte zu reduzieren.

In der Ausgangsstellung ist der Nadelhalter 8 mit einem starren vorderen Abschnitt 9 auf den Anschluss 12 des Katheteranschlussstückes 1 aufgesetzt. An seinem vorderen Abschnitt 9 weist der Nadelhalter 8 ein Griffstück 3 auf. Neben dem vorderen starren Abschnitt 9 weist der Nadelhalter 8 einen dem erstgenannten gegenüber liegenden, ebenfalls starren hinteren Abschnitt 10 sowie einen die beiden starren Abschnitte 9 und 10 verbindenden, mittleren, flexiblen Abschnitt 11 auf. Der mittlere Abschnitt 11 des Nadelhalters 8 ist, wie in Fig. 2 zu erkennen ist, durch zwei Schenkel 16 gebildet, welche über Filmscharniere in sich und gegenüber den vorderen bzw. hinteren Abschnitten 9 und 10 beweglich sind. In der in den Fig. 1 und 2 gezeigten Ausgangsstellung sind die Schenkel 16 auseinander gespreizt und gefaltet, so dass der vordere Abschnitt 9 und der hintere Abschnitt 10 des Nadelhalters 8 einander axial benachbart sind. Die Punktionsnadel 5 ist mit ihrem dem Punktionsende 14 gegenüber liegenden, hinteren Ende 17 in dem hinteren Abschnitt 10 des Nadelhalters 8 festgelegt. Dadurch befindet sich in der Ausgangsstellung die Punktionsnadel 5 in ihrer maximal in den Verweilschlauch 4 vorgeschobenen Position, in welcher die Punktionsspitze 14 über das Abgabeende 15 des Verweilschlauches 4 vorsteht. Die Punktionsnadel 5 ist dabei durch die dem Verweilschlauch 4 am Anschluss 12 gegenüber der Umgebung abdichtende Dichtung 7 hindurchgeführt. Auf diese Weise kommt die erfindungsgemäße Punktionsanordnung mit einer einzigen Dichtung 7 aus. Die Punktionsnadel 5 läuft im Innern des Verweilschlauches 4 und ist im Bereich des Katheteranschlussstückes 1 entsprechend dem Verlauf des Verweilschlauches 4 ebenfalls gekrümmt geführt. In dem Nadelhalter 8 erstreckt sich die Punktionsnadel 5 im wesentlichen in Flucht zu dem hinteren, dem Anschluss 12 zugewandten Abschnitt des Verweilschlauches 4.

Zum Anbringen der Punktionsanordnung muss der Patient zunächst die Schutzkappe 6 entfernen und die Schutzfolie von der Klebeplatte 2 abziehen. Nun drückt er die Punktionsanordnung senkrecht auf die zu punktierende Stelle seines Leibes, wobei die Punktionsnadel 5 durch die Haut in das darunter liegende Gewebe eindringt und zugleich Raum für den ebenfalls eindringenden Verweilschlauch 4 schafft. Für diese Tätigkeit wird der Patient die Punktionsanordnung an dem Griffstück 3 greifen und halten.

Ist die Punktionsanordnung in dieser Weise auf den Körper aufgesetzt, die Punktionsstelle durchstochen und das Katheteranschlussstück 1 mittels der Klebeplatte 2 fixiert, muss der Patient die Punktionsnadel 5 aus dem Verweilschlauch 4 entfernen. Hierzu werden zunächst die Schenkel 16 des mittleren Abschnitts 11 des Nadelhalters 8 aus ihrer in der Ausgangsstellung (Fig. 2) gespreizten Position zusammengedrückt, wodurch sich der hintere Abschnitt 10 vom vorderen Abschnitt 9 des Nadelhalters 8 entfernt (vgl. Fig. 3 und 4). Dadurch wird die mit ihrem hinteren Ende 17 im hinteren Abschnitt 10 des Nadelhalters 8 fixierte Punktionsnadel 5 sukzessive aus dem Verweilschlauch 4 in Richtung des Anschlusses 12 durch die Dichtung 7 zurückgezogen. Der dabei im Inneren des Verweilschlauches 4 entstehende Unterdruck bewirkt, dass Körperflüssigkeit (Blut, Gewebeflüssigkeit) in das Abgabeende 15 des Verweilschlauches 4 eindringt und diese eingesaugt wird. Durch nicht näher dargestellte Rasteinrichtungen an den Schenkel 16 können diese in der gestreckten Position gegeneinander verrastet werden, um zu verhindern, dass die Punktionsnadel 5 erneut in den Verweilschlauch 4 eindringt. Der Verweilschlauch 4 ist nun frei durchgängig und aufgrund des oben beschriebenen Unterdruckes nahezu vollständig mit Körperflüssigkeit geflutet. Ein separates Fluten oder Entlüften des Verweilschlauches 4, wie es bei bekannten Punktionsanordnungen aus dem Stand der Technik erforderlich ist, kann entfallen. Der Nadelhalter 8 ist in seiner Längenerstreckung derart ausgebildet, dass bei gestreckten Schenkeln 16 die Punktionsnadel 5 sich mit ihrer Punktionsspitze 14 geschützt im Inneren des vorderen Abschnittes 9 des Nadelhalters 8 liegt. Der Nadelhalter 8 kann nun durch Ziehen an dem Griffstück 3 von dem Anschluss 12 gelöst und abgezogen und anschließend sicher entsorgt werden.

Es ergibt sich nun die in Fig. 5 gezeigte Situation, in der das Katheteranschlussstück 1 mit seinem Anschluss 12 zum Anschluss eines entsprechenden Gegenstückes 18 einer Versorgungsleitung 19 freiliegt. Der Verweilschlauch 4 liegt sicher im Gewebe des Patienten, ihr Durchgang ist geflutet und für die Zufuhr einer Medikamentenversorgung frei.

Nicht zuletzt aus der voranstehenden Beschreibung des Ausführungsbeispieles ergibt sich, dass die erfindungsgemäße Punktionsanordnung durch die spezielle Führung der Punktionsnadel 5 im Inneren des Verweilschlauches 4 über dessen gesamten Verlauf und aus dem Katheteranschlussstück 1 über dieselbe Dichtung 7 heraus die gewünschten Vorteile ergibt. Der Verweilschlauch kann verglichen mit bekannten Varianten mit deutlich geringerem Durchmesser ausgeführt werden; ein separates Fluten bzw. Entlüften des Verweilschlauches 4 vor dem eigentlichen Einsatz ist nicht erforderlich, die Handhabung dieser Punktionsanordnung ist insgesamt vereinfacht.

### Bezugszeichenliste

- 1: Katheteranschlussstück
- 2: Klebeplatte
- 3: Griffstück
- 4: Flexibler Verweilschlauch
- 5: Punktionsnadel
- 6: Schutzkappe
- 7: Dichtung
- 8: Nadelhalter
- 9: vorderer Abschnitt
- 10: hinterer Abschnitt
- 11: mittlerer Abschnitt
- 12: Anschluss
- 13: Austrittsöffnung
- 14: Punktionsende
- 15: Abgabeende
- 16: Schenkel
- 17: hinteres Ende
- 18: Gegenstück
- 19: Versorgungsleitung

## Patentansprüche

1. Punktionsanordnung mit einem flexiblen Verweilschlauch (4) zum Anschließen an eine medizinische Versorgungsleitung (19) mit einem an einer Einstichstelle auf der Hautoberfläche eines Patienten fixierbaren Katheteranschlussstück (Hub) (1), welches einen Anschluss (12) zum Anlegen der Versorgungsleitung und eine Austrittsöffnung (13) für den flexiblen Verweilschlauch (4) aufweist, und mit einem eine Punktionsnadel (Stilett) (5) aufweisenden Nadelhalter (8), **dadurch gekennzeichnet, dass** der flexible Verweilschlauch (4) im Inneren des Katheteranschlussstückes (1) angeordnet und mit einem Abgabeende (15) aus der Austrittsöffnung (13) heraus geführt ist, wobei das dem Abgabeende (15) gegenüberliegende Ende des flexiblen Verweilschlauches (4) im Inneren des Katheteranschlussstückes (1) im Bereich des Anschlusses (12) liegt und von der Umgebung des Katheteranschlussstückes (1) durch eine Dichtung (Septum) (7) getrennt ist und wobei der Nadelhalter (8) die in einen Durchflusskanal des Verweilschlauches (4) einführbare Punktionsnadel (5) an einem dem spitzen bzw. scharfen Punktionsende (14) derselben gegenüberliegenden Ende (17) fixiert und in einer Ausgangstellung der Punktionsanordnung so an dem Anschluss (12) des Katheteranschlussstückes (1) von diesem lösbar angeordnet ist, dass die Punktionsnadel (5) die Dichtung (7) durchdringt und bis über das Abgabeende (15) hinaus in dem Durchflusskanal des Verweilschlauches (4) geführt ist, und dass die Austrittsöffnung (13) auf einer in Gebrauchsstellung auf der Patientenoberfläche aufliegenden Unterseite des Katheteranschlussstückes (1) angeordnet ist und dass die Punktionsnadel durchgangslos gefertigt ist.

2. Punktionsanordnung nach Anspruch 1, **dadurch gekennzeichnet, dass** das Katheteranschlussstück (1) im wesentlichen flach ausgebildet ist, wobei der Verweilschlauch (4) schräg zu einer Ebene, in der das im wesentlichen flache Katheteranschlussstück (1) liegt, auf der Unterseite des Katheteranschlussstückes (1) aus der Austrittsöffnung (13) herausragt und im Inneren des Katheteranschlussstückes (1) umgelenkt und in der Ebene geführt ist, und dass der Anschluss (12) in Flucht mit dem dem Abgabeende (15) gegenüberliegenden Ende des Verweilschlauches (4) ausgerichtet ist.

3. Punktionsanordnung nach einem der vorhergehenden Ansprüche, **dadurch gekennzeichnet, dass** der Nadelhalter (8) einen starren, in der Ausgangsstellung an dem Anschluss (12) angeordneten vorderen Abschnitt (9), einen starren hinteren Abschnitt (10), in welchem das dem Punktionsende (14) gegenüberliegende Ende (17) der Punktionsnadel (5) festgelegt ist, und einen diese Abschnitte (9, 10) verbindenden flexiblen mittleren Abschnitt (11) aufweist, wobei der flexible Abschnitt (11) durch zwei unter Belassung eines Abstandes einander gegenüber liegende, mit den starren Abschnitten (9, 10) vorzugsweise über Filmscharniere flexibel verbundene Schenkel (16) gebildet ist, welche in der Ausgangsstellung derart nach außen verformt sind, dass der vordere (9) und der hintere (10) Abschnitt einen minimalen Abstand zueinander aufweisen.

4. Punktionsanordnung nach Anspruch 3, **dadurch gekennzeichnet, dass** die Schenkel (16) Rasteinrichtungen aufweisen, mit denen sie in einer gestreckten Position, in welcher der vordere (9) und der hintere (10) Abschnitt einen maximalen Abstand zueinander aufweisen, gegeneinander verrastet und so in dieser Position gesichert werden können.

5. Punktionsanordnung nach einem der Ansprüche 3 oder 4, **dadurch gekennzeichnet, dass** die Längenabmessungen der Punktionsnadel (5) und der Abschnitte (9, 10, 11) des Nadelhalters (8) so gewählt sind, dass bei gerade gestreckten Schenkeln (16) das Punktionsende (14) der Punktionsnadel (5) innerhalb des Nadelhalters (8) liegt.

6. Punktionsanordnung nach einem der vorhergehenden Ansprüche, **dadurch gekennzeichnet, dass** der Nadelhalter (8) ein Griffstück (3) aufweist.

7. Punktionsanordnung nach einem der vorhergehenden Ansprüche, **dadurch gekennzeichnet, dass** die Dichtung (7) vorperforiert ist.

8. Punktionsanordnung nach einem der vorhergehenden Ansprüche, **dadurch gekennzeichnet, dass** das Katheteranschlussstück (1) auf einer zum Aufsetzen auf die Hautoberfläche des Patienten vorgesehenen Unterseite eine selbstklebende Schicht (2) aufweist.

9. Punktionsanordnung nach einem der vorhergehenden Ansprüche, **dadurch gekennzeichnet, dass** sie in einem original versiegelten Zustand eine über das Abgabeende (15) des Verweilschlauches (4) sowie das Punktionsende (14) der Punktionsnadel (5) aufgesetzte Schutzkappe (6) aufweist.

10. Punktionsanordnung nach einem der vorhergehenden Ansprüche, **dadurch gekennzeichnet, daß** der Verweilschlauch (4) im Innern des Katheteranschlussstückes (1) zur Änderung seiner Verlaufsrichtung in entgegengesetzten Krümmungsrichtungen doppelt gebogen verläuft.

## Claims

1. Puncture system with a flexible self-retaining tube (4) for connection to a medical supply line (19) with a catheter connecting piece (1) fixable at a puncture point on the skin surface of a patient and which has a connection (12) for the application of the supply line and an outlet (13) for the flexible self-retaining tube (4), and with a needle holder (8) having a puncture needle (stiletto) (5), **characterized in that** the flexible self-retaining tube (4) is located in the interior of the catheter connecting piece (1) and is led out of outlet (13) with a delivery end (15), the end of the flexible self-retaining tube (4) opposite to the delivery end (15) being located within the catheter connecting piece (1) in the vicinity of connection (12) and is separated from the environment of the catheter connecting piece (1) by a seal (septum) (7) and in which the needle holder (8) fixes the puncture needle (5) insertable into a flow duct of the self-retaining tube (4) at an end (17) opposite to the pointed or sharp puncture end (14) and in a starting position of the puncture system is so detachably positioned at the connection (12) of the catheter connecting piece (1) that the puncture needle (5) penetrates the seal (7) and is passed beyond the delivery end (15) in the flow duct of the self-retaining tube (4) and that the outlet (13) is located on an underside of the catheter connecting piece (1) resting on the patient surface in the use position and that the puncture needle is made in passageless manner.

2. Puncture system according to claim 1, **characterized in that** the catheter connecting piece (1) is constructed in a substantially flat manner, the self-retaining tube (4) being inclined to a plane in which the substantially flat catheter connecting piece (1) is located, projects out of the outlet (13) on the underside of the catheter connecting piece (1) and in the interior of the latter is deflected and guided in the plane, and that the connection (12) is oriented in alignment with the end of the self-retaining tube (4) opposite to delivery end (15).

3. Puncture system according to one of the preceding claims, **characterized in that** the needle holder (8) has a rigid front portion (9) located on connection (12) in the starting position, a rigid rear portion (10) in which is fixed the end (17) of puncture needle (5) opposite to the puncture end (14), and a flexible central portion (11) linking said portions (9, 10), the flexible portion (11) being formed by two facing, whilst leaving a spacing, legs (16) flexibly connected to the rigid portions (9, 10), preferably by means of film hinges and which in the starting position are outwardly deformed in such a way that the front portion (9) and rear portion (10) have a minimum mutual spacing.

4. Puncture system according to claim 3, **characterized in that** the legs (16) have locking devices with which in an extended position, where the front portion (9) and rear portion (10) have a maximum mutual spacing, they are locked together and can in this way be secured in this position.

5. Puncture system according to one of the claims 3 or 4, **characterized in that** the linear dimensions of the puncture needle (5) and portions (9, 10, 11) of needle holder (8) are chosen in such a way that with the legs (16) straightened, the puncture end (14) of puncture needle (5) is located within needle holder (8).

6. Puncture system according to one of the preceding claims, **characterized in that** needle holder (8) has a grip (3).

7. Puncture system according to one of the preceding claims, **characterized in that** seal (7) is preperforated.

8. Puncture system according to one of the preceding claims, **characterized in that** the catheter connecting piece (1) is provided on an underside for placing on the skin surface of the patient with a self-adhesive coating (2).

9. Puncture system according to one of the preceding claims, **characterized in that** in an original sealed state it has a protective cap (6) placed over the delivery end (15) of self-retaining tube (4) and puncture end (14) of puncture needle (5).

10. Puncture system according to one of the preceding claims, **characterized in that** the self-retaining tube (4) in the interior of the catheter connecting piece (1) is twice curved in opposite curvature directions for changing its running direction.

## Revendications

1. Dispositif de ponction avec un tuyau flexible permanent (4) pour le raccordement à une conduite d'alimentation médicale (19) avec un raccord de cathéter (course) (1) pouvant être fixé en un point de piqûre sur la surface de peau d'un patient, qui présente un branchement (12) pour l'application de la conduite d'alimentation et un orifice de sortie (13) pour le tuyau flexible permanent (4), et avec un porte-aiguille (8) présentant une aiguille de ponction (stylet) (5), **caractérisé en ce que** le tuyau flexible permanent (4) est disposé à l'intérieur du raccord de cathéter (1) et est guidé par une extrémité de distribution (15) à la sortie de l'orifice de sortie (13), l'extrémité, opposée à l'extrémité de distribution (15), du tuyau flexible permanent (15) étant disposée à l'intérieur du raccord de cathéter (1) dans la zone du branchement (12) et étant séparée de l'environnement du raccord de cathéter (1) par un joint (septum) (7) et le porte-aiguille (8) fixant l'aiguille de ponction (5) pouvant être introduite dans un canal d'écoulement du tuyau permanent (4) sur une extrémité (17) faisant face à l'extrémité de ponction (14) pointue ou aiguisée et étant disposé dans une position de du dispositif de ponction sur le branchement (12) du raccord de cathéter (1) de façon détachable de cette pièce, de telle sorte que l'aiguille de ponction (5) traverse le joint (7) et est guidée jusqu'au-delà de l'extrémité de distribution (15) dans le canal d'écoulement du flexible permanent (4), et **en ce que** l'orifice de sortie (13) est disposé sur un côté inférieur, reposant dans la position d'utilisation sur la surface du corps du patient, du raccord de cathéter (1) et **en ce que** l'aiguille de ponction est fabriquée sans passage.

2. Dispositif de ponction selon la revendication 1, **caractérisé en ce que** le raccord de cathéter (1) est conçu sensiblement plat, le tuyau permanent (4) dépassant de l'orifice de sortie (13) sur le côté inférieur du raccord de cathéter (1) en biais par rapport à un plan dans lequel est disposé le raccord de cathéter (1) sensiblement plat et étant dévié à l'intérieur du raccord de cathéter (1) et guidé dans le plan, et **en ce que** le branchement (12) est orienté en alignement avec l'extrémité, faisant face à l'extrémité de distribution (15), du flexible permanent (4).

3. Dispositif de ponction selon l'une quelconque des revendications précédentes, **caractérisé en ce que** le porte-aiguille (8) présente un tronçon (9) avant rigide, disposé dans la position de départ sur le branchement (12), un tronçon (10) arrière rigide, dans lequel l'extrémité (17), faisant face à l'extrémité de ponction (14), de l'aiguille de ponction (5) est fixée, et un tronçon (11) central, flexible, reliant ces tronçons (9, 10), le tronçon (11) flexible étant formé par deux branches (16) se faisant face en laissant une distance entre elles reliées de façon flexible aux tronçons (9, 10) rigides de préférence par des charnières à film, lesquelles branches sont déformées dans la position de départ vers l'extérieur de telle sorte que le tronçon avant (9) et le tronçon arrière (10) présentent un espacement minimum entre eux.

4. Dispositif de ponction selon la revendication 3, **caractérisé en ce que** les branches (16) présentent des dispositifs d'encliquetage avec lesquels elles peuvent être encliquetées les unes par rapport aux autres dans une position étirée, dans laquelle le tronçon avant (9) et le tronçon arrière (10) présentent un espacement maximum entre eux, et peuvent ainsi se verrouiller dans cette position.

5. Dispositif de ponction selon l'une quelconque des revendications 3 et 4, **caractérisé en ce que** les dimensions longitudinales de l'aiguille de ponction (5) et les tronçons (9, 10, 11) du porte-aiguille (8) sont choisis de telle sorte que, dans le cas de branches (16) étirées en alignement, l'extrémité de ponction (14) de l'aiguille de ponction (5) est située à l'intérieur du porte-aiguille (8).

6. Dispositif de ponction selon l'une quelconque des revendications précédentes, **caractérisé en ce que** le porte-aiguille (8) présente une poignée (3).

7. Dispositif de ponction selon l'une quelconque des revendications précédentes, **caractérisé en ce que** le joint (7) est préperforé.

8. Dispositif de ponction selon l'une quelconque des revendications précédentes, **caractérisé en ce que** le raccord de cathéter (1) présente une couche (2) autocollante sur un côté inférieur prévu pour la pose sur la surface de la peau du patient.

9. Dispositif de ponction selon l'une quelconque des revendications précédentes, **caractérisé en ce qu'**il présente dans un état scellé à l'origine un capot de protection (6) posé au-dessus de l'extrémité de distribution (15) du tuyau permanent (4) et de l'extrémité de ponction (14) de l'aiguille de ponction (5).

10. Dispositif de ponction selon l'une quelconque des revendications précédentes, **caractérisé en ce que** le tuyau permanent (4) est agencé avec un double coude à l'intérieur du raccord de cathéter (1) pour la modification de son sens de tracé dans des directions de courbure opposées.
